# EUROPEAN PATENT SPECIFICATION

(11) **EP 3 780 002 B1**
(45) Date of publication and mention of the grant of the patent: **17.07.2024**
(21) Application number: 20189384.9
(22) Date of filing: 23.11.2011
(51) Int. Cl.: G16H 50/30

(54) **SYSTEMS AND METHODS TO ASSESS COGNITIVE FUNCTION**
SYSTEME UND VERFAHREN ZUR BEURTEILUNG DER KOGNITIVEN FUNKTION
SYSTÈMES ET PROCÉDÉS POUR ÉVALUER LA FONCTION COGNITIVE

(30) Priority: 24.11.2010 US 41718910 P
(43) Date of publication of application: 17.02.2021
(62) Divisional of application: 11843900.9
(73) Proprietor: Clinical Ink, Inc., Horsham PA 19044 (US)
(72) Inventor: SEVERSON, Joan Marie, Cedar Rapids, IA Iowa 52242 (US); COSMAN, Joshua, Daniel, Iowa City, IA Iowa 52245 (US); MERICKEL, Michael Bruce, Jr., Coralville, IA Iowa 52241 (US)
(74) Representative: Pons

(56) References cited:
- WO-A1-2010/045356
- WO-A2-2004/073485
- WO-A2-2007/147083
- WO-A2-2009/152216
- US-A1- 2005 053 904

## Description

### BACKGROUND

There are many individuals that would like to assess their cognitive function, either compared to self over time or compared to others at particular times and/or over time. Additionally, there are a number of entities that would benefit from being able to assess the cognitive function of individuals. Allowing these individuals and entities to assess cognitive function compared to others with a particular chosen characteristic, such as age group, income level, health status, etc. would be particularly helpful. The prior art does not provide any system that allows individuals and these entities to quickly, reliably, and cost-effectively assess cognitive function compared to a current, relevant, and statistically-meaningful sample of data (e.g., other individuals having the same or similar demographic characteristics).

Systematic normative and clinical data updates are important to keep up with changes in test performance in the population. However, the efficiency, sharing, and integration limitations in the current testing methods do not permit such data collection. More specifically, persons of skill in the art are presented with a significant problem of obtaining reliable data from suitable subjects. It should be appreciated that, for example, subjects that have previously taken a test may provide responses that skew the results of normative data. For example, if the subject is overly familiar with the test, the data may be useless or even harmful to the collective data set to be analyzed. In such a case, the test performance could be deemed as measuring training effect rather than measuring a subject's cognitive abilities or functions. In general, using such data may be frowned upon, and may be considered by many as junk science.

Currently available cognitive assessment tools include traditional paper and pencil tests as well as computerized and internet-based tests. Despite the variety of developed testing tools and test administration methods, the limitations described above remain for several reasons.

Using traditional cognitive assessment methods, a pool of subjects representative of a population of interest is selected. The statistically relevant sample of subjects is then tested to develop a normed performance metric. Individuals taking the same test at a later time can then be compared to this historical pre-selected reference group. Maintaining the currency of the normed performance metric is incredibly time-consuming and expensive because new sample groups must be repeatedly identified and tested with the results incorporated. Identifying new sample groups is time consuming and expensive. Also, the actual test administration is time consuming and expensive as well. These costs typically involve millions of dollars and thousands of hours of work for each normed data set. For example, in a clinical setting typically used in the prior art, administration, analysis, and reporting of a typical battery of cognitive assessment tests may cost approximately $2,000 to $3,000 per individual. Because of the costs associated with maintaining currency of the data, the normed data sets are often outdated and do not reflect current societal norms. However, it is often critical, or at least very important, that the data obtained for analysis is reliable data that can be validated, and strict adherence to established procedures for including selection of individuals for testing, test administration, and the like is followed at great expense.

There are also drawbacks associated with traditional paper and pencil tests and computerized and internet-based tests. For example, paper and pencil tests generally must be individually administered by a trained clinician. This makes the testing procedures inefficient and time consuming.

Moreover, despite training, it is inevitable that differences in testing procedures will occur. These differences can be very subtle, relating to the speed of presentation of instructions, breaks in phrasing, voice quality, or voice intonation and modulation, direction of the examiner's gaze at the materials, frequency and appropriateness of eye contact, etc. Other factors can include subtle changes in verbal instructions, familiarity and facility in the manipulation of test materials.

Additional differences during test administration include the clinician's appearance, gender, and ethno-cultural background. Even factors such as height, hair color, and attire introduce additional variation. There can also be the divergent influence of interpersonal factors relating to the quality of rapport between the clinician and the subject.

Tests that do not require individual administration still require time for scoring and scoring of these tests is not always performed consistently across test-takers.

Computerized and/or internet-based cognitive assessment tools address some of the problems discussed above, yet still suffer many of the same limitations of the traditional tests. Overall, test administration is more firmly standard, detailed timing parameters are collected, and scoring accuracy is improved. Limitations remain, however, with regard to low throughput due to individual administration and inefficient data collection and sharing. For example, it may be difficult and expensive to determine which individuals should perform the test. Also, many currently available computer tests have an additional limitation related to the interface between the subject and the computer. For example, different keyboards often have different response times, which may degrade the quality of reaction time data obtained therefrom. For example, display and operating systems have different refresh rates and resolutions, and operating system performance may influence the performance metrics, and thus, the reliability of the data. Accordingly, these limitations may require test reliability validation for each computing device configuration and each specific interface environment, which is generally infeasible or unpractical. Further, self-administration of cognitive assessment tests is not generally accepted for providing reliable data which can be validated. The prior art does not provide any assessment platform that is easy to use to obtain reliable data and cost effective, to the contrary, the prior art systems and methods are generally difficult to implement and administer, and are cost ineffective.

US 2005/053904 A1 (SHEPHARD JENNIFER [US] ET AL) 10 March 2005 (2005-03-10) discloses a computerized method and system for assessing cognitive function for individuals.

### SUMMARY

The invention is defined by the method of independent claim 1 and the system of independent claim 13. Optional aspects of the invention are provided in the dependent claims.

Additional features and advantages of the disclosed method and apparatus are described in, and will be apparent from, the following Detailed Description and the Figures. Examples/aspects/embodiments not according to the invention as defined by the claims are present for illustration purposes only.

### BRIEF DESCRIPTION OF THE FIGURES

To complement the description being made and in order to aid towards a better understanding of the characteristics of the invention, in accordance with a preferred example of practical embodiment thereof, a set of drawings is attached as an integral part of said description wherein, with illustrative and non-limiting character, the following has been represented:
Figure 1 illustrates a block diagram of an example screen layout for a cognitive assessment test on a portable touchscreen personal computing device, according to an example embodiment of the present invention.
Figure 2 is a flowchart illustrating an example cognitive assessment method, according to an example embodiment of the present invention.
Figure 3 is a detailed block diagram of an example cognitive assessment system, according to an example embodiment of the present invention.
Figure 4 is a flowchart illustrating an example cognitive assessment method, according to an example embodiment of the present invention.

### PREFERRED EMBODIMENTS OF THE INVENTION

Example embodiments disclosed herein provide self-administered cognitive assessment tools on portable touchscreen personal computing devices. The exemplary assessment tools disclosed in detail herein are based, in part, on reaction time measures to computer-based stimuli. In an example embodiment, user performance metrics are automatically uploaded to a database or databases at an assessment computer. The data is dynamically normed through continuous uploading of user performance metrics. This approach allows for the development of norms based on self-reported behavior and demographics and allows for the creation of a large sample size of varying demographics and behavior that can influence cognitive function. Also, administration of cognitive assessment tests is time consuming and expensive, so self-administration of tests saves a great deal of time and money. As noted above, for example, in a clinical setting typically used in the prior art, administration, analysis, and reporting of a typical battery of cognitive assessment tests may cost approximately $2,000 to $3,000 per individual. On the contrary, using the presently disclosed system and method, the administration, analysis, and reporting of a typical battery of cognitive assessment tests may cost approximately $24. These cost savings, along with savings of cost on test selection and/or subject selection, provide for expenses that are orders of magnitude lower than the prior art methods, which allows for far greater data throughput. Further, self-administration may provide higher quality cognitive assessment data in some respects. For example, the individual taking the test finds the touchscreen user interface extremely simple and intuitive, so the thought process is not interfered with by thinking about manipulating a mouse, a keyboard, navigating a paper answer sheet, or the like. There may be little to no learning of the user interface required.

The benefits of self-administration may save a great deal of expense in generating a normed performance metrics and validating data, however, the disclosed method of self-administration may also require the use of data which is unreliable and unsuitable for validation. For example, an individual which takes a test while intoxicated will provide bad data. Accordingly, using self-administration for collecting cognitive assessment data as disclosed herein flies in the face of many of the teachings of the prior art. However, as disclosed herein, the presently disclosed portable touchscreen personal computing device provides an intuitive and easy to use user interface which overcomes these problems, even when the data received is anonymous data that would generally be unsuitable for use. The presently disclosed system and method provide an intuitive mobile assessment platform via portable touchscreen personal computing devices, such as commonplace devices like the iPad^{®}, which advantageously allows for increased throughput of cognitive test performance, through increased accessibility, increased usability, a more isolated analysis of cognitive ability, decreased training for test administration, decreased administration costs, decreased or eliminated subject selection costs, etc. Moreover, costs and difficulties associated with performing cognitive function assessment may be significantly reduced, and quick, reliable, and cost-effective cognitive function assessment with respect to a current, relevant, and statistically-meaningful data set that includes validated performance metrics may be more easily provided.

The database(s) with performance metrics may be queried by third parties evaluating the effects of demographic variables on cognitive performance. Moreover, third parties can use the data to evaluate cognitive function against performance of various tasks. Product developers may use the data to help create products that are cognitively appropriate for a targeted demographic.

The exemplary tests chosen for inclusion with the present disclosure assess cognitive function by measuring abilities related to attention, memory, executive function and processing speed.

As used herein, "attention" refers to an individual's ability to focus on information that is relevant to a task at hand while ignoring information that is not relevant to the particular task.

As used herein, "memory" refers to an individual's ability to biologically store information in the short-term or long-term.

As used herein, "executive function" describes an individual's ability to plan, solve problems, and initiate appropriate and inhibit inappropriate actions for a task at hand.

As used herein, "processing speed" refers to how quickly an individual is able to perceive and interpret sensory information.

As used herein, "cognitive function" refers to the overall ability with which individuals are able to perceive, remember and act on information in the environment, and can also be used to refer to one of the core mental abilities mentioned above. For example, the term can be used in either of the following contexts: "cognitive training leads to improved cognitive functioning" or "attention is considered a core cognitive function."

As used herein, "cognitive baseline" refers to a stable state of cognitive functioning at a given point in time. The baseline can act as a point of reference for assessing cognitive decline, improvement, or maintenance over time and/or due to e.g., aging or pathological conditions, exposure to an event or chemical, etc. For example, assessment tools may be used as a screening instrument for traumatic brain injury (TBI), including mild TBI. For example, a military application may provide for an assessment test within one hour of a blast exposure, which is coupled with a physiological measurement procedure.

Attention, memory, executive function, and processing speed were chosen as aspects of mental processing to support creation of a cognitive baseline because they can be considered "core" cognitive functions, and as a result provide the most useful means of assessing cognitive baselines in both neuro logically normal and neuro logically impaired individuals. In particular, these functions have been implicated in the performance of real-world tasks affected by cognitive decline, such as driving or problem solving, and as a result the measurement of these particular functions provides predictive value in the assessment of cognitive ability. Each of the tests listed below taps one or more of these functions, using both accuracy and reaction time measures. Because of the emphasis of these tasks on reaction-time measures, they can provide a sensitive assessment of cognitive function and changes therein. It should be appreciated that different cognitive functions may be used or not used, and likewise, one or more of the exemplary cognitive functions may not be used for determining a cognitive baseline and/or assessing cognitive function.

Moreover, the example systems described herein include a suite of tests that primarily employ reaction-time measures, providing a continuous variable measure of cognitive performance that is more sensitive to subtle changes in cognitive functioning than traditional neuropsychological tests. Lees et al, (2010) Translating cognitive neuroscience to the driver's operational environment: A neuroergonomics approach. American Journal of Psychology, 123, 391-411. Continuous variable measures provide greater sensitivity to detecting changes within and across individuals because they allow for a continuous measure of cognitive function. For example, most assessment tests used in a clinical setting are relatively easy, use subjective scoring methods (i.e., clinicians rate and score responses), and interpret these scores in a nominal (impaired vs. unimpaired) or ordinal (as in IQ tests) manner. This works for describing gross deficits in cognitive functioning like those experienced after brain damage, but works less well for measuring subtle changes in cognitive function in normal, healthy adults because most healthy adults would have little difficulty performing the tests. Accordingly, these tests do not discern cognitive changes in normal high functioning individuals. The tests described herein using reaction-time measures, however, allow the detection of minute differences in cognitive function, based on differences in how long it takes a user to respond to stimuli under differing stimulus conditions.

The disclosed example embodiments allow the creation of a personal cognitive baseline for an individual. The baseline can be used to compare the individual's performance to that of his or her "peers" or "cohorts" (used interchangeably herein) based on chosen characteristics that make them peers, or to compare the individual's performance under varying circumstances (e.g., before and after taking medication) or for comparison over time, such as to measure cognitive decline or improvement relative to one's self or one's peers.

Chosen characteristics to define peers can include, without limitation, characteristics such as gender; birth year; ethnicity (e.g., Hispanic/Latino, American Indian or Alaska Native, Asian, Black or African American, Native Hawaiian or Pacific Islander, White); highest education (e.g., high school, some college, bachelor's degree, graduate degree); income per year (e.g., 0-9,999; 10,000-24,999; 25,000-49,999; 50,000-74,999; 75,000+); health (e.g., measured as times per week performing exercise (strenuous, moderate, and mild)); leisure hours per week spent: playing video games, reading, surfing the web, watching television; hours per day spent sleeping; how many languages the individual can read and write; and whether in the past two years the individual has learned a new language or musical instrument.

Examples of appropriate tests may include, without limitation:
Visual Short-term memory (VSTM). In the visual short-term memory task, individuals are briefly presented with four color patches presented at the center of the screen and are asked to remember the colors. Following a short delay, a single color patch is shown and the individual is asked whether the color was one of those presented or not. For example, on a given trial an individual can be briefly presented with color patches in blue, red, green and yellow and asked to remember them. If they were then shown the color purple, they would respond "no match" because the color purple was not in the presented and remembered set. This test measures the ability to remember visual information in the short-term.

Spatial Working Memory (SPWM). In the spatial working memory task one to three objects are briefly flashed on the screen and then disappear, and individuals are asked to remember the locations of each of the objects. After a brief delay, a single object appears on the screen and the participant responds to whether or not the object is in the same location as one of the objects being remembered. This task measures the ability to remember visuospatial information in the short-term.

N-back. In the N-back task individuals are presented with a continuous stream of letters at the center of the screen and are asked to respond whether the letter presented on the current trial matches the one presented on the previous trial. For example, an individual can see the letter H followed by the letter T, and would be asked to respond to whether the H and T match identity or not (the case in this example). This test measures how well participants can hold and manipulate information in short-term memory. In another version of this task, individuals are presented with a continuous stream of letters at the center of the screen and are asked to respond whether the letter presented on the current trial matches the one presented two trials ago.

Stroop Task. In the Stroop task individuals are asked to name the color of a written word presented at the center of the screen as quickly as possible. The word can either be a color-word (e.g., the word red written in either green or red) or a non-color word (e.g., the word cat written in red). The ability to focus attention is assessed by seeing how much an incorrect color/word combination (e.g., the word red written in green) slows an individual's reaction time. This task provides a measure of how well an individual can control attention and executive function processes.

Attention Blink. In the Attentional Blink task an individual views a stream of letters presented rapidly at the center of the screen and is asked to search the stream for either one or two pre-defined target letters. On trials in which there are two targets, detecting the first target interferes with the ability of an individual to detect the second target, and the extent of this interference is used to assess attention function.

Task Switch. In the task-switch task, individuals see a digit (e.g., 1-10) at the center of the screen, and the digit appears on a color patch. Depending on the color of the patch, the individual has to respond to either the parity (e.g., high vs. low) of the number or whether the number is odd or even. Importantly, on each trial the color patch is either the same color as the previous trial, resulting in participants performing the same task from trial to trial, or a different color than the previous trial, resulting in a switch in the task. For example, on a given trial an observer can see the number two on a pink color patch. On this trial, the individual would perform the parity judgment task. On the following trial, if the color patch stays the same the individual would continue to perform the parity task. However, if the color patch changes color, this signals that the individual should switch and perform the odd/even task on this trial. This task measures the ability to rapidly switch tasks, a subset of executive function.

Trails A&B. In the Trails task, individuals are to connect dots in sequence as quickly as possible using their finger. In trails A, individuals are asked to connect dots 120 in sequence. In trails B, individuals are asked to connect many more dots or dots 110 and A- J in sequence, alternating between numbers and letters. This test measures how quickly individuals can search for and sequentially process information from the within a category (Trails A) or between categories (Trails B). The Trails test measures of attention and executive function.

Flanker Task. In the flanker task, individuals are presented with a display containing several objects. One of the objects, the target, is always presented at the center of the screen, and participants are asked to identify which of two target types the item is. The target is flanked on both sides by distractor objects that are either identical to the target on a given trial or not. For example, participants can view a display containing multiple arrows. One arrow, the target, will be presented at the center of the screen and participants' task is to report whether the arrow is pointing to the left or to the right. This arrow is surrounded on both sides by arrows that are pointing in either the same or different directions. This task assesses how well individuals can focus attention on relevant and ignore irrelevant visual information, providing a measure of attention and executive function.

Visual Search Task. In the Visual Search Task, individuals are presented with an array of objects and are asked to find a target object as quickly as possible. For example, an individual can be told to search for a particular color box with a gap in the top or bottom, and report the location (top or bottom) of the gap. This task assesses how quickly an individual can find and identify basic visual information, a subset of attention function.

Perceptual Motor Speed (PMS). In this task, individuals are presented with a schematic face and are asked to press a button as soon as possible in response to a happy face, and withhold their response to a sad face. The ability to withhold a response to sad faces provides a measure of executive function, and the speed with which responses to happy faces are made provides a measure of processing speed.

Basic Processing Speed. In this task, individuals monitor a blank screen, and after a variable delay a small circle appears at the center of the screen. Participants are asked to press a button as quickly as possible when they see the circle appear. This task measures basic visual processing speed.

Digit Span. In this task observers see strings of two to eight numbers, and are asked to remember their identities and their order. After the strings are removed from the screen, participants need to type as many of the numbers as they can remember. This test provides a measure of verbal short-term memory.

Additional tests known to those of ordinary skill in the art can also be used. As will be understood by those of ordinary skill in the art, the systems and methods disclosed herein can utilize all tests described above or a subset of these tests in various combinations. The embodiments can also use varying numbers of these tests, typically, anywhere from 1 to 20 tests, including 1 test, 2 tests, 3 tests, 4 tests, 5 tests, 6 tests, 7 tests, 8 tests, 9 tests, 10 tests, 11 tests, 12 tests, 13 tests, 14 tests, 15 tests, 16 tests, 17 tests, 18 tests, 19 tests or 20 tests.

The suite of tests is administered on a personal computing device with a touch-activated screen, such as an iPad^{®}, iPhone^{®} or other similar device. For this reason, the tests described above are adapted for use with a touchscreen user interface. In the example of an iPad^{®} most tests can be adapted for providing a response button on each end of the screen. The individual taking the test can hold the iPad^{®} on both ends, using his or her thumbs to select the answer selection on the left of the screen or the answer selection on the right of the screen.

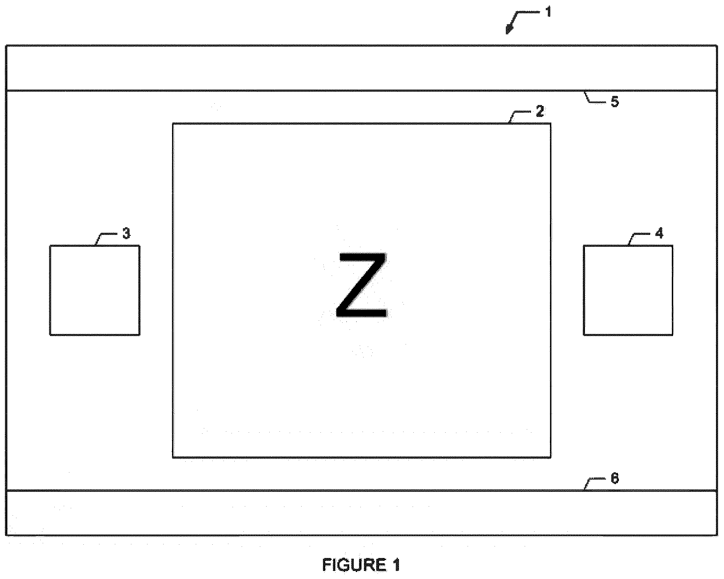

Figure 1 illustrates a block diagram of an example screen layout for a cognitive assessment test on a portable touchscreen personal computing device, for example, the N-Back test discussed above. The example screen layout includes a stimuli area 2, a left side selection button 3, a right side selection button 4, a header 5, and a footer 6. The stimuli area 2 provides the stimuli for the individual to react to, for example, letters (e.g., "Z"), numbers, colors, shapes, and the like are provided according to the particular cognitive test being administered at a given time. The stimuli provided in the stimuli area 2 may be centrally located, peripherally located, and/or move around the stimuli area 2 in an ordered or random fashion. Also, for example, the stimuli area 2 may be provided to cover a smaller or larger area, including spanning the entire screen layout 1. Then, the individual reacts to the stimuli provided in the stimuli area 2 by pressing either the left side selection button 3 or the right side selection button 4. The left side selection button 3 and the right side selection button 4 are touchscreen buttons provided on the display screen of the personal computing device. It should be appreciated that the individual may be able to change settings or customize the screen to move the left side selection button 3 and/or the right side selection button 4, for example up or down along the edge of the display for ease of use, comfort, and the like. Further, it should be appreciated that touchscreen left and right side selection buttons 3, 4 are not required, and that other configurations of touchscreen buttons, including more or less buttons, different positioning, different sizes and/or shapes, etc. Also, it should be appreciated that no touchscreen buttons may be required for providing response, for example, as with the Trails test when the individual traces a finger or stylus around on the stimuli area 2, as discussed above. A header 5 may be provided with information relating to the cognitive test being taken, the current time, battery life, wireless connection information, a quit button, etc. A footer 6 may also be provided with information, such as a progress bar, and functional buttons (e.g., Begin Practice, Begin Test, Continue, Back). It should be appreciated that the screen layout 1 is merely an illustrative example and may be modified in a variety of ways for usability, aesthetics, branding, and the like.

Further, for example, in the Visual Search Test, the left side selection could be "Bottom" and the right side selection could be "Top." In the Flanker Test utilizing pointing arrows, the left side selection could be "Left" and the right side selection could be "Right." Similarly, in the N- back test, the left side selection could be "No Match" and the right side selection could be "Match." A progress-line and icon near the bottom of the screen (e.g., in the footer 6) may be used to provide the user with an indication of remaining queries in the particular practice session or assessment.

Instructions and explanations of the test that are easy to understand are also provided to increase the likelihood that the tests are self-administered properly. Examples for a subset of the described tests as they could be administered on a portable touchscreen personal computing device are described below:

### General Introductory Instructions before Starting Assessments:

In order to get an accurate measure of your cognitive function, make sure you are sitting somewhere quiet where you won't be interrupted or distracted during the test.

Hold your device with your hands on both sides of the screen, and use your thumbs to respond.

Press the Continue key to continue.

### Exemplary Screens for Administration of VSTM

### VSTM General Introduction & Instructions:

This test measures your visual memory. You will see a set of 4 colored boxes flash on the screen, and you must remember the color of each box.

### (Diagram)

After a short delay, you will be shown another colored box and be asked if it matches the color of one of the boxes you saw in the previous set.

If the square matches one in the previous set, press the MATCH key with your left thumb. If the square doesn't match one in the previous set, press the NO
MATCH key with your right thumb.

The first block of trials will be practice. Try as best you can to remember the colors of the four boxes, and respond as accurately as possible.

### VSTM Practice

### VSTM Assessment Instructions:

This task will be identical to the one you performed in the practice. Remember to respond as accurately as possible.

### Exemplary Screens for Administration of SPWM

### SPWM General Introduction & Instructions:

This test measures your spatial memory. You will see a set of 2 or 3 black dots, and you will try to remember the locations of these dots.

### (Diagram)

After a short delay, you will be shown a red dot and will have to respond whether the red dot matches the location of one of the black dots.

If the location of the red dot matches the location of one of the black dots, press the MATCH key with your left thumb. If the location of the red dot doesn't match the location of one of the black dots, press the NO MATCH key with your right thumb.

The first block of trials will be practice. Try as best you can to remember the locations of the black dots, and respond as accurately as possible.

### SPWM Practice

### SPWM Assessment Instructions:

This task will be identical to the one you performed in the practice. Remember to respond as accurately as possible.

### Exemplary Screens for Administration of Nback

### Nback- 1 General Introduction & Instructions:

This test measures your working-memory. A sequence of letters will appear at the center of the screen, and you will have to respond to whether the current letter matches the one you saw just before it.

If the current letter matches the previous letter, press the MATCH key with your left thumb. If not, press the NO MATCH key with your right thumb. The first block of trials will be practice. Try to respond as accurately as possible.

### Nback- 1 Practice

### Nback- 1 Assessment Instructions:

This task will be identical to the one you performed in the practice. Remember to respond as accurately as possible.

### Nback-2 Practice Instructions:

This part of the test will be similar to what you just did, but this time you will have to respond to whether the current letter matches the one you saw TWO letters before it.

If the current letter matches the letter TWO letters before it, press the MATCH key with your left thumb. If not, press the NO MATCH key with your right thumb.

The first block of trials will be practice. Try to respond as accurately as possible.

### Nback-2 Practice

### Nback-2 Assessment Instructions:

This task will be identical to the one you performed in the practice. Remember to respond as accurately as possible.

### Exemplary Screens for Administration of Stroop

### Stroop General Introduction & Instructions:

This test measures how well you can block out distraction. You will see colored words presented at the center of the screen. Your task will be to respond to the COLOR of the words.

Press the button matching the COLOR of the word. For example if you saw the following, you would press the green key. (Picture of color word, red printed in green)

The first block of trials will be practice. Try respond as quickly and accurately as possible.

### Stroop Practice

### Stroop Assessment Instructions:

This task will be identical to the one you performed in the practice. Remember to respond as quickly and accurately as possible.

### Exemplary Screens for Administration of Blink

### Blink General Introduction & Instructions:

This test measures how quickly you can pay attention to things across time. You will see a stream of letters presented rapidly at the center of the screen, and you will watch the stream for a target letter.

### Blink Target 1 Practice Instructions:

In the first part of the test, you will watch the stream of letters at the center of the screen and look for the RED letter.

When the stream ends, you will be asked to respond to the identity of the red letter, which will always be a B,G, or S. Respond using the B, G, or S buttons at the bottom of the screen.

Please respond as accurately as you can.

### Blink Target 1 Practice

### Blink Target X Practice Instructions:

In this part of the test, you will watch the stream of letters at the center of the screen and look for the letter X.

On some trials, there will be an X in the stream. In other trials, there won't be an X. When asked if you saw an X in the stream, you will press the YES button if you saw an X, and the NO button if you did not.

Remember to respond as accurately as you can.

### Blink Target X Practice

### Blink Combined Practice Instructions:

In this part of the test, you will look for BOTH the red letter and the letter X on each trial.

At the end of the stream, you will first respond to the identity of the RED letter, and then you will respond to whether or not you saw an X. Remember to respond as and accurately as you can.

### Blink Combined Practice

### Blink Combined Assessment Instructions:

This block will be exactly like the practice you just performed.

Remember to respond as quickly and accurately as possible.

### Exemplary Screens for Administration of Task Switching

### Task Switch General Introduction & Instructions:

This test measures how quickly you can switch between two different tasks. First, you will practice each task individually. Then we will combine the tasks and measure how well you can switch between them.

### Task 1 Practice Instructions:

On each trial, there will be a box with a number inside of it. Your task will be to decide whether the digit is HIGHER or LOWER than the number 5. Use your left thumb to respond HIGH or LOW. If the number is higher than 5, press the HIGH key. If the number is lower than 5, press the LOW key. Please respond as quickly and accurately as you can.

### Task 1 Practice

### Task 2 Practice Instructions:

Just as before, on each trial there will be a box with a number inside of it. For this task, you will decide whether the digit is ODD or EVEN. Use your right thumb to respond, and press the ODD button if the number is odd and the EVEN button if the number is even.

Please respond as quickly and accurately as you can.

### Task 2 Practice

### Task Switch Combined Practice Instructions:

Now you will be asked to switch between the two tasks you just practiced. The digit will appear inside of either a blue or pink square. If the square is BLUE, perform the high/low task using your left thumb. If the square is PINK perform the odd/even task using your right thumb.

The color of the square will change unpredictably, so be ready to perform both tasks. This block of trials will be practice.

Please respond as quickly and accurately as you can.

### Combined Task Practice

### Task Switch Combined Assessment Instructions:

This block will be exactly like the practice you just performed. Remember to respond as quickly and accurately as possible.

### Exemplary Administration of Trails

### Trails A General Introduction & Instructions:

This task measures how quickly you can search for information in space. The task will be similar to connect the dots, and you will have to use your finger to sequentially connect dots numbered 1 -20, as quickly as possible. To make the task more difficult, you will not be able to lift your finger once you begin, otherwise you will be forced restart the task.

Trails a Practice with hypothetical practice performance.

Optional Instructions for Trails B.

Trails practice based on second instruction set.

In certain embodiments, completion of an assessment can generate an end screen stating, for example:
You have completed the test. Press the "Results" button to see your score.

Following responses during practice sessions and/or actual assessment, the individual taking the assessment can be given feedback regarding the correctness of his or her responses. In certain embodiments, this feedback is only provided during the practice phase of the assessment. For example, the feedback may state "Correct" or "Incorrect" or "No Response Detected" after a response is provided.

Initially, the individual may be presented with a home screen that allows the user to choose his or her already-existing profile or to create a new profile. An individual may create a user profile by walking the user through a series of questions eliciting different types of demographic data. During profile creation the systems and methods disclosed herein will ask particular questions of the user. In some embodiments, defined response options can be provided for selection (e.g., a dropdown list). In other embodiments, questions can be openended with no defined response options provided. Some embodiments may employ a mix of these question types. Particular embodiments can also allow a user to enter personal characteristics not particularly requested.

Next, the individual can choose tests from the available suite (e.g., Blink, Flanker, N- Back, PMS, Posner, Speed, SPWM, Stroop, Task Switching, Trails A & B, Visual Search, VSTM). Following selection by the individual, instructions such as those described above are provided for the chosen test and practice questions are provided. Then, the individual takes the test. Answer selections are made directly on the touchscreen without the need for an intermediate device and performance (e.g., percent correct and response time) is recorded. Scores and user performance data are uploaded to the assessment computer database which can sort, filter and aggregate information for transmission back to the personal computing device. Based on communication with the assessment computer database, a report of the data can be created on the personal computing device or created from the database and downloaded to the personal computing device. The mean data can be represented via a hot-cold graphical plane, and individual scores can be plotted above or below the mean. The mean data may continually change to reflect the population the individual is interested in comparing himself or herself to. Baselines for cohorts may be continually updated as individuals self-administer the suite of tests and additional performance data is collected.

Individuals within the dataset may be compared to different groups or cohorts based on selected demographic characteristics. For example, the individual may request that test results be analyzed against cohorts of the same age (e.g., 47 years old). Alternatively, the individual may request an age around his or her own age such as 45 to 50 years old. Alternatively, the user could request comparison to a different peer group such as 20 year olds, or those that are more or less physically active and/or socially active. It should be appreciated that studies have shown that physical activity appears to have an influence on cognitive function (e.g., individuals with higher rates of physical activity have higher cognitive function scores in specific areas of cognition). Alternatively, the systems and methods disclosed herein can utilize default cohort creations based on one or more chosen characteristics. The same is true when the database is queried by a third party. Individual's cohort groups within the database may change based on selected demographics and how these demographics are defined by the party submitting the query (e.g., the systems and methods described herein allow the creation of "retrospective cohort groups").

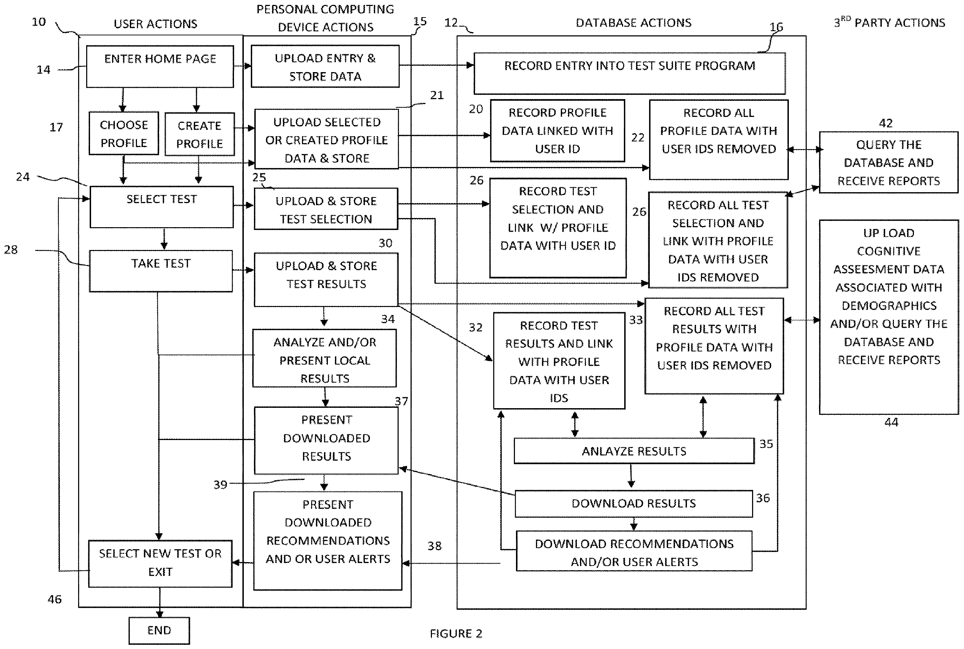

Figure 2 is a flowchart illustrating an example cognitive assessment method, according to an example embodiment of the present invention. The exemplary method utilizes a portable touchscreen personal computing device 10 on which an individual can perform cognitive assessment tasks and an assessment computer 12 that can, without limitation, store, aggregate, filter, analyze and sort stored data using a database.

Upon initiation of use, a user enters a home page 14. Optionally, the methods described herein can upload 15 and record the entry of a user into the home page into the database 16. At the home page, the user can select an existing profile 17 or choose to create (or edit) a profile 18. The user (or the entity directing the user to perform the cognitive assessments described herein) can decide whether the user's profile is linked with a unique identifier of the particular user 20 or is uploaded independently of a unique identifier with demographic information only 22 after which the profile data can be uploaded 21 and recorded 20, 22. A unique user identifier can be created from any suitable data unique to the user including, without limitation, a password, a fingerprint, a profile name, a face-print, etc. Additionally, some personal computing devices, such as mobile phones can be tied to a user by default and can require no separate user identifier.

Following creation and/or selection of a user profile, the user can select a cognitive assessment task 24. Optionally, the methods disclosed herein may upload 25 and store the task selection into the database 26. This information may be useful to assess percentage of aborted tests, tests chosen but not taken, etc. in conjunction with or independently of demographic data to assess ability or willingness of users to complete the test.

Following task selection, a processor can run the assessment tools based on the user's request. Users can be presented with test instructions and take the selected tests 28 as described in more detail above. All test results are uploaded to the database 30 with results linked to a particular user also linked with the user's previous uploaded data 32. Results not linked with user IDs are also stored with associated demographic information of the user profile 33. Storage of the data can be accomplished in any suitable manner, including distributing the data across different storage media on the same or different computing devices, for example, across multiple hard drives in a RAID server, or multiple computing devices in a cloud storage system.

Results related to number of queries answered correctly, incorrectly or not responded to along with information relating to timing of test taking optionally can be generated locally by the user's personal computing device 34. The user's personal computing device 10 can also store previous performance specific to the user profile and display changes against previous performance 34. The assessment computer 12 may analyze results 35 for populating the database. Analyzed data can be tabulated and downloaded by the database 36. The user may be presented with downloaded results 37. Additionally, the assessment computer 12 can provide current comparative data against peers or cohorts defined by shared demographic characteristics, which may include, for example, behavioral characteristics, medical history characteristics, educational characteristics, and training experience characteristics. A user's activity history or a group of users' activities can be analyzed to generate particular reports. The reports can be in the form of text, charts, graphs, animations, and/or illustrations. The reports can include data related to any aspect of the user's activity history. For example, the reports can track the user's level of performance of specific cognitive tasks over a period of time, showing how the level of the user's performance has varied based on the passage of time, or in relation to the user's level of performance of other cognitive tasks, the user's previous performance on the same task, the performance of others with a shared or shared demographic characteristics of the user, etc.

Aggregating user information can involve, for example, updating cumulative totals, updating cumulative totals for the same cognitive task, averaging performance over time for individual assessment tasks and/or groups of tasks, etc... For example, the user information can be aggregated with any data already in the user's activity history in the database.

Based on the results that are generated and/or presented to the user, the personal computing device 10 and/or assessment computer 12 can generate recommendations or user alerts based on comparisons to previous results 38. The assessment computer 12 can also generate recommendations or user alerts based on comparisons to the particular user's cohorts 40.

In steps 35 and 38 the user's informational history can be used to evaluate the user's cognitive functions, and to make recommendations of programs for the user to use. For example, through step 35 and 38, the level of performance of the user can be used to recommend to the user programs that will help the user improve at cognitive tasks at which they show deficiencies, sharpen the user's ability to perform specific cognitive tasks that the user is already good at but wishes to be better at, etc. These programs can be computer or non-computer based (e.g., recommendations for diet or physical activity). The recommendation can include programs the user should interact with, a schedule providing for when and how long the user should interact with certain programs, and/or goals or checkpoints for the user's level of performance of certain cognitive assessment tasks, etc. These methods additionally permit comparison to a user's own individual performance over time as compared to self and cohorts allowing even more sensitive monitoring of the individual's cognitive abilities over time. Alerts/recommendations can also suggest visiting a physician. Accordingly, the information may become part of the individual's medical record as a screening instrument for physicians. Methods disclosed herein can also utilize cognitive data and associated demographic characteristics uploaded from other external sources 42.

Additionally, external subscribers can access the "all results" data in the database (i.e. all data uploaded and linked with demographic information but user IDs never present or removed) and query this data to assess demographic influences on cognitive abilities. This access can be based on a per query fee or can be provided based on pre-purchased time allotments. Alternatively, external subscribers could pay a flat fee for access for a defined period of time such as an hour, day, week, month, year or perpetual. Subscribers may also be given free access to the "all results" data.

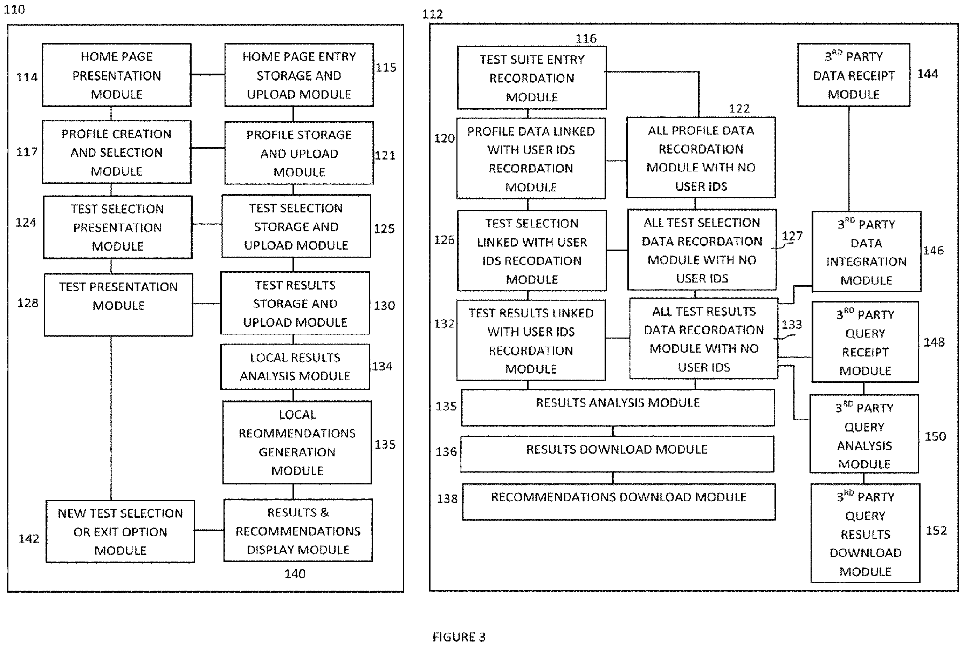

Figure 3 is a detailed block diagram of an example cognitive assessment system, according to an example embodiment of the present invention. The personal computing device 110 can be any suitable combination of hardware and software that allows the individual to perform the cognitive assessments on a touch-activated screen. The personal computing device 110 includes a processor, a touchscreen, and a communications device. In an example embodiment, the personal computing device 110 may include eye tracking and/or speech recognition technology. The suite of assessment tools described herein may be stored on the personal computing device 110 or may reside on external media that can be accessed by the personal computing device 110.

The processor can be any general or special purpose microprocessor, depending on the nature and uses of the personal computing device 110, suitable for executing, and allowing the user to interact with the suite of tests described herein.

The communications device can be any suitable device for allowing the personal computing device 110 to send data to and receive data from the assessment computer 112, which includes a database. For example, the communications device can be, without limitation, an 802.1 Ix protocol based wireless (Wi-Fi) card, an Ethernet device, a modem, a Bluetooth module, an IR link, etc. The personal computing device 110 can make use of more than one communications device, for example, a personal computing device can have a Wi-Fi card and a Bluetooth module. The communications device can be connected to the personal computing device 110 in any suitable manner, for example, using wired or wireless connections.

The assessment computer 112 database may embody any type of suitable data structure and may be provided for using any suitable combination of hardware and software of assessment computer 112 to suitably allow for the storage and analysis of the data stored within. For example, the database may be provided on one or more computers which comprise the assessment computer 112, including for example a database server, a server cluster, and/or one or more computers. It should be appreciated that the assessment computer 112 may be provided, for example, on multiple computers of a distributed computing network, and the database may or may not be distributed amongst multiple separate computers. The assessment computer 112 includes a processor and a communications device. The assessment computer 1 12 database can receive cognitive assessment performance and demographic characteristics uploaded from a plurality of portable touchscreen personal computing devices through the personal computing devices communications device(s).

For simplicity of the figures, particular hardware such as processors and communications devices, which are known be persons of skill in the art, are not explicitly depicted in Figure 3. Instead, this figure depicts a personal computing device 110 including functional modules such as a home page presentation module 114, a profile creation and selection module 117, a test selection presentation module 124, a test presentation module 128, a home page entry storage and upload module 115, a profile storage and upload module 121, a test selection storage and upload module 125, a test results storage and upload module 130, a local results analysis module 134, a local recommendation generation module 135 (in the context of Figure 3, "recommendations" include recommendations and "user alerts" as described above), a results and recommendations display module 140 and a new test selection or exit option module 142. Figure 3 also depicts an assessment computer 112 including a test suite entry recordation module 116, a profile data linked with user IDs recordation module 120, an all profile data recordation module without user IDs 122, a test selection linked with user IDs (and associated profile data) recordation module 126, an all test selection data recordation module with no user IDs (with test selections linked to other profile information) 127, a test results linked with user IDs (and associated profile data) recordation module 132, an all test results data recordation module with no user IDs (with test results linked to other profile information); a results analysis module 135, a results download module 136 and a recommendations download module (which can also create recommendations based on analyzed results). Assessment computer 112 can also include a third party data receipt module 144, a third party data integration module 146, a third party query receipt module 148, a third party query analysis module, 150 and a third party query analysis download module 152. It should be appreciated that the assessment computer 112 may include one or more databases to store and access data for the assessment of cognitive function as disclosed herein. Based on the preceding descriptions and figures, one of ordinary skill in the art understands the various possible system architectures of the systems disclosed herein and how the various modules may interconnect and function.

Computers and computer systems described herein can include operatively associated computer-readable media such as memory for storing software applications used in obtaining, processing, storing and/or communicating data. It can be appreciated that such memory can be internal, external, remote or local with respect to its operatively associated computer or computer system. Memory can also include any means for storing software or other instructions.

In general, computer-readable media can include any medium capable of being a carrier for an electronic signal representative of data stored, communicated or processed in accordance with embodiments disclosed herein. Where applicable, method steps described herein can be embodied or executed as instructions stored on a tangible computer-readable medium or media.

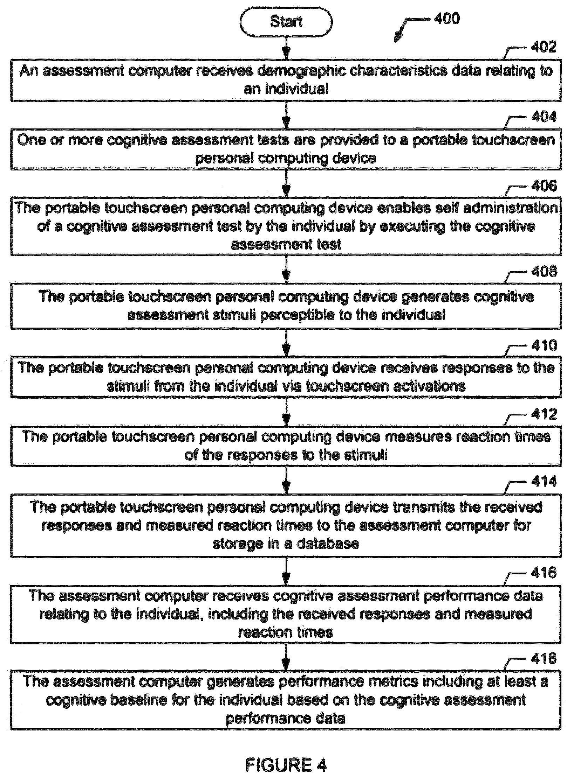

Fig. 4 is a flowchart of an example process 400 for assessing cognitive function. Although the process 400 is described with reference to the flowchart illustrated in Fig. 4, it will be appreciated that many other methods of performing the acts associated with the process 400 may be used. For example, the order of many of the blocks may be changed, certain blocks may be combined with other blocks, and many of the blocks described are optional.

An assessment computer receives demographic characteristics data relating to an individual (block 402). For example, the individual may fill out a survey with information about the individual's age, race, health, sleep habits, income level, exercise level, etc., which is transmitted to the assessment computer from the individual's iPad^{®}. One or more cognitive assessment tests are provided to a portable touchscreen personal computing device (block 404). The iPad^{®} downloads several cognitive assessment tests, which the individual can choose to perform.

The portable touchscreen personal computing device enables self-administration of the cognitive assessment test by the individual by executing the cognitive assessment test (block 406). For example, the iPad^{®} allows the user to self-administer cognitive assessment tests. The portable touchscreen personal computing device generates cognitive assessment stimuli perceptible to the individual (block 408). For example, the iPad^{®} provides on its screen the Flanker test. The portable touchscreen personal computing device receives responses to the stimuli from the individual via touchscreen activations (block 410). For example, the individual presses a touchscreen button on the left or right sides of the iPad^{®} screen with his thumbs based on the direction of arrows provided by the Flanker test. Also, in an example embodiment, a touchscreen may not be required, and a touch activation may be replaced with eye tracking and/or speech recognition. The portable touchscreen personal computing device measures reaction times of the responses to the stimuli (block 412). For example, the iPad^{®} measures the time for each response from the individual. The portable touchscreen personal computing device transmits the received responses and measured reaction times to the assessment computer for storage in a database (block 414). For example, the iPad^{®} uploads the left and right selections, and the corresponding measured response times, to the assessment computer for storage and analysis.

The assessment computer receives cognitive assessment performance data relating to the individual, including the received responses and measured reaction times (block 416). For example, data indicating the specific test taken, the time of the administration of the test, each selection response, and the corresponding measured response times, is received at the assessment computer, and stored for immediate or future analysis. The assessment computer generates performance metrics including at least a first cognitive baseline for the individual based on the cognitive assessment performance data (block 418). For example, a cognitive baseline for the individual is created or updated based on the responses, reaction times, etc. Also, normed performance metrics may be generated based on cognitive assessment performance data obtained from many different individuals. Further, generated performance metrics may be validated using statistical analysis.

It should also be appreciated that user performance metrics may be generated at the personal computing device (e.g., an iPad^{®}) and/or at the assessment computer. For example, prior to administration of a cognitive assessment test, the portable touchscreen personal computing device may receive a set of normative values which may be used to provide a real-time comparison or analysis of the individual's performance. Standard normed performance metrics may be general or specific (e.g., tailored to certain demographic characteristics of the individual). It should be appreciated that a subset of the analysis that may be performed at the assessment computer may be performed on portable touchscreen personal computing device. Also, for example, an iPad^{®} may receive an update to a stored set of normative values each time the internet is accessed. Accordingly, the individual may receive real-time feedback based on performance metrics from a portable touchscreen personal computing device even when no internet connection is available.

Benefits of the systems and methods disclosed herein may include creation of societal cognitive baselines that can be used by clinicians and researchers similar to those provided by blood pressure, blood sugar, or cholesterol baselines; an ability for individuals or entities to track performance of an individual against previous performance of the individual or against the individual's cohorts quickly and reliably by having the individual self-administer the tests at various time points or events; reduces costs compared with psychological tests that must be administered by a trained clinician; reduces costs and collects reliable data due to the absence of an intermediate answer selection device (such as a keyboard that requires mapping a user's response to the keyboard which in turn requires more practice before giving tests, and more errors in testing). The database of the assessment computer can also be queried by individuals, entities or researchers to generate reports based on questions presented about the data. These benefits are described below in the context of real-world examples of how the systems and methods disclosed herein could be used.

### Uses for the Military

The systems and methods disclosed herein may allow reliable cognitive assessments to be performed quickly, reliably and easily by a number of individuals and different entities that previously could not perform such assessments without undue burden and expense. For example, embodiments disclosed herein allow the military to assess cognitive function of military personnel at the beginning of military service, periodically during the service career, prior to deployment, during deployment, any time after a mild or traumatic brain injury and/or on return from deployment. Military personnel could be given an individual personal computing device to carry throughout their service or could be provided with a personal computing device at the particular times the military would like to evaluate an individual or group of individuals' cognitive performance. For example, an assessment test may be performed within one hour of a blast exposure and coupled with a physiological measurement procedure (e.g., heart rate variability).

Accordingly, this application of the embodiments disclosed herein provides numerous benefits including allowing service personnel to self-administer cognitive assessment tests, perform testing in remote locations, perform testing with commercial off-the-shelf computing hardware, test a large number of individuals at particular times, aggregate test performance metrics overtime, and link test performance metrics with characteristics such as military training, service record, and/or medical records.

### Uses in Clinical Research - Environmental Simulators

Embodiments described herein may allow clinical research groups to link an individual or groups cognitive function to simulator-based performance metrics. For example, those interested in examining how individuals or groups of individuals cognitively perform in real world activities such as driving, frequently evaluate driver performance and behavior through controlled simulation based technologies (e.g., vehicle simulator, flight simulator, bicycling simulator, military platform simulator. Simulator based performance metrics, such as number of errors committed during a driving scenario, lane deviations, collisions, speeding violations) could be automatically linked to cognitive performance metrics and demographics gathered by the disclosed invention.

### Uses in Clinical Research - Substance Exposure

Embodiments described herein may allow clinical research groups to link an individual or groups cognitive functions to exposure to chemical substances. For example, the systems and methods disclosed herein could be used to assess the cognitive function of a patient or research subject before, after and/or during exposure to chemical substance ingestion, exposure, and or administered through medical treatment (e.g., pharmaceuticals, alcohol, nicotine, industrial bi- products, food-based nutrients, etc.).

### Uses For Occupational or Rehabilitation Therapists and Medical Practitioners

Embodiments described herein may allow occupational or rehabilitation therapists or medical practitioners to link a patient or research subjects' cognitive function to rehabilitative treatment or therapy.

Accordingly, this application of the embodiments disclosed herein provide numerous benefits including automated linking of cognitive function of a patient or research subjects cognitive response to rehabilitative treatment or therapy protocols or regimes. This allows reporting of a patient or research subject's performance measures at a single visit and over longitudinal measurements of performance as well as the linking of cognitive performance metrics to digital medical records.

### Uses for Employers and Human Resources Departments

Embodiments described herein may allow employers and human resources departments to test an employee's cognitive functions prior to and during the course of employment. Accordingly, this application of the embodiments disclosed herein provides numerous benefits including employee self-testing with automated scoring and reporting of an employees performance measures at the beginning of employment and over longitudinal measurements of performance and the potential to link cognitive assessments to additional digital employee testing instruments. Performance metrics of individuals can be used to create a baseline of multiple domains of cognitive function to determine areas of strength and weaknesses in an employee's ability. These measures can be used to evaluate areas of service that personnel can be assigned to or trained in that would be appropriate to enhance work performance.

### Uses in Athletics

Embodiments described herein may allow athletic personnel to test a player's cognitive function prior to and following sports-related head injuries and/or over the course of a season with or without head injury. This application allows the ability to obtain pre-injury baselines and post-injury assessments in cognitive function in amateur and professional athletes, as well as the ability to administer said tests in remote locations. Additionally, the absence of brain injury, the effects of regular exercise on cognitive performance can be assessed.

### Uses by an Individual

Embodiments described herein may allow an individual to track cognitive performance over time against self and/or peers based on chosen or predetermined demographic characteristics. Individuals can also identify areas of deficiency to inform areas for cognitive function or ability training. Improvement based on such training can also be monitored.

### Uses by Parents & Teachers

Parents and teachers can also utilize the systems and methods disclosed herein to assess the cognitive development of children. Performance on the provided test of suites can be compared to classroom performance or other factors to assess performance against peers or to uncover potential disconnections between cognitive ability and performance in other areas. This may allow parents and teachers to understand the abilities of particular children to create appropriate learning environments.

### Uses to Assess Effects of Environment on Cognitive Function

In certain embodiments part of the user profile can provide information relating to the individual's geographic location. In personal devices that do not have GPS or a similar location system, the user can enter a zip code of residence or present location. In devices that do have GPS or a similar location system, current location can be tracked and data can reflect residence location and current location. This data could be especially useful for researchers hoping to understand the effects of location on cognitive function. Locations could be correlated with environmental factors such as urban vs. rural; proximity to a power supply; proximity to a museum; proximity to a university; land value; transport of hazardous materials through or near a location; ambient noise levels; state or county of residence; access to public transportation; crime statistics; local liquor regulations; etc. In essence, researchers could use the systems and methods disclosed herein to uncover correlations between cognitive function and any other metric that can be associated with a geographic location.

### Uses to Assess Fatigue

Fatigue plays a role in many work-related accidents and traffic accidents. Moreover, fatigue can decrease attention, memory and other cognitive functioning, leading to costly mistakes. The systems and methods used herein can be used to create a baseline of cognitive performance of cohorts when alert and when impaired due to fatigue. Individual performance when alert compared to when fatigued can also be utilized when alert performance data is available for an individual. When individual alert data for an individual is not available, the individuals' performance can be compared to a cohort cognitive baseline. This use of the systems and methods disclosed herein can be useful to assess the fatigue level of shift workers, drivers, residents in hospitals, surgeons before beginning an operation, athletes before being put into a game, etc. In certain embodiments, police may carry portable touchscreen personal computing devices as described herein to assess the fatigue level of drivers. If drivers fall below a certain level, this could provide a basis for citation - driving while fatigued.

### Uses in Technology Development

Embodiments described herein may allow product or technology research and development groups to link individuals or groups cognitive function to usage behavior metrics. Accordingly, this application of the embodiments disclosed herein provide numerous benefits including filtering of demographic data and automated linking of cognitive function of individuals and groups to technology usage behavior metrics, support of technology design to meet the cognitive abilities of identified demographic groups and inform design of technology to meet cognitive abilities to market demographics.

For example, similar to the concept of ergonomics that designs physical objects to fit the physical characteristics of an intended user, systems and methods disclosed herein allow the design of objects to match the cognitive abilities of intended users.

One example is use with video game design and/or marketing. Automated linking of demographics and cognitive function performance metrics would provide information to game designers and marketing firms that would be similar to providing physiological measures of demographic groups to companies that are designing running shoes. The game designers and/or marketers can then design products that fit the cognitive abilities they would like to develop for or market to.

Particularly, designing game mechanics involves understanding how a player is able to respond to a scenario, speed and response to stimuli within a game or game level. Current methods used to "tweek" game design to optimize players experience include play testing, some physiological response measures (such as GSR or EEG) and visual maps of player movement throughout an environment. Utilizing the systems and methods disclosed herein in designing games would increase the likelihood of game dynamics matching the cognitive abilities of the target audience, increasing enjoyment and resulting revenue generated by the game.

Automated linking of demographics data to cognitive function performance metrics could also be used to improve user satisfaction with personal devices such as mobile devices. Mobile device and computer interface device makers aim to develop interfaces that "match" the cognitive abilities of their target users. Understanding differences in memory and or ability to filter visual distracters would benefit user interface design for more mature markets, such as cell phone markets. This concept could also be extended to automobile manufactures and dashboard controls and displays, cockpit displays and other areas that can utilize human factors and cognitive function metrics to inform design.

The phrase "portable touchscreen personal computing devices" has been used throughout this disclosure, which is meant to encompass iPads^{®}, iPhones^{®} and other similar devices, for example, including gaming consoles (Wii, Xbox, Nintendo, etc.), gaming console control devices (accelerometer, computer vision, biometric interfaces, auditory, KINECT, etc.), mobile phones and other devices with hand-held touchscreens and/or with physical buttons for answer selection.

The terms "a," "an," "the" and similar referents used in the context of describing the exemplary embodiments (especially in the context of the following claims) are to be construed to cover both the singular and the plural, unless otherwise indicated herein or clearly contradicted by context. Recitation of ranges of values herein is merely intended to serve as a shorthand method of referring individually to each separate value falling within the range. Unless otherwise indicated herein, each individual value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein is intended merely to better illuminate the exemplary embodiments and does not pose a limitation on the scope of the exemplary embodiments otherwise claimed. No language in the specification should be construed as indicating any non-claimed element essential to the practice of the exemplary embodiments.

It will be appreciated that all of the disclosed methods and procedures described herein can be implemented using one or more computer programs or components. These components may be provided as a series of computer instructions on any conventional computer-readable medium, including RAM, ROM, flash memory, magnetic or optical disks, optical memory, or other storage media. The instructions may be configured to be executed by a processor, which when executing the series of computer instructions performs or facilitates the performance of all or part of the disclosed methods and procedures.

It should be understood that various changes and modifications to the example embodiments described herein will be apparent to those skilled in the art. Such changes and modifications can be made within the scope of the appended claims, which define the invention.

## Claims

1. A computerized method for assessing cognitive function, the method comprising:
- receiving, at an assessment computer, demographics data relating to an individual, including information relating to portable touchscreen personal computing device geographic location;
- correlating a portable touchscreen personal computing device geographic location with at least one environmental factor;
- providing, by a first 802.11 x protocol communication, at least one cognitive assessment test to the portable touchscreen personal computing device, which, when executed by the portable touchscreen personal computing device enables self administration of the at least one cognitive assessment test by the individual by:
∘ generating cognitive assessment stimuli perceptible to the individual;
∘ receiving responses to the stimuli from the individual via touchscreen activations;
∘ measuring reaction times of the responses to the stimuli; and
∘ transmitting the received responses and measured reaction times to the assessment computer for storage in a database;
- receiving, by a second 802.11 x protocol communication, first cognitive assessment performance data relating to the individual, said first cognitive assessment performance including the received responses and measured reaction times;
- generating performance metrics including at least a first cognitive baseline for the individual based at least on the first cognitive assessment performance data; and
- comparing, by the assessment computer, at least a first cognitive baseline with performance data of other individuals with common demographic characteristics based on the at least one environmental factor.

2. The method of claim 1, further comprising a step of storing in a database accessible by the computer data relating to at least one of time reaction values, stimuli, performance metrics, demographics, tests, and behavior.

3. The method of claim 1, wherein the stimuli are at least one of visual stimuli, tactile stimuli, and audio stimuli.

4. The method of claim 1, wherein measuring reaction times is based on an appearance of the cognitive assessment stimuli and the receiving of the responses via the touchscreen activations.

5. The method of claim 1, further comprising:
providing feedback to the individual relating to the first cognitive assessment performance data.

6. The method of claim 1, further comprising:
identifying the individual, wherein the individual's user profile is linked with a unique identifier of the individual; and
associating first cognitive assessment performance data with the individual's user profile.

7. The method of claim 1, wherein the generation of performance metrics includes: storing the first cognitive assessment performance data including at least results related to a number of queries answered correctly, incorrectly, and not responded to, along with information relating to the measured reaction times.

8. The method of claim 1, further comprising comparing the first cognitive assessment performance data with second cognitive assessment performance data from previous performance of the individual, which is stored in the database.

9. The method of claim 1, wherein the first cognitive baseline is automatically updated each time the individual performs a cognitive assessment test.

10. The method of claim 1, wherein the individual self-reports all of the demographics data relating to the individual.

11. The method of claim 1, wherein the portable touchscreen personal computing device generates user performance metrics based on the first cognitive assessment performance data and including a comparison to normed performance metrics stored on the personal computing device, and displays the user performance metrics in real-time to the individual.

12. The method of claim 1, wherein the portable touchscreen personal computing device receives further responses to the stimuli from the individual via at least one of eye tracking and speech recognition.

13. A system for assessing cognitive function, the system comprising:
∘ an 802.11 x protocol based wireless card;
∘ a tangible computer readable medium storing cognitive assessment performance data associated with an individual, environmental data associated with the individual, demographic characteristic data, and performance metrics; and
∘ at least one processing device operably coupled to the tangible computer readable medium, the at least one processing device configured to:
- receive, at an assessment computer, demographics characteristic data and the environmental data;
- provide, through the 802.11 x protocol based wireless card, at least one cognitive assessment test to a portable touchscreen personal computing device, which, when executed by the portable touchscreen personal computing device enables self administration of the at least one cognitive assessment test by the individual by:
▪ generating cognitive assessment stimuli perceptible to the individual;
▪ receiving, through the 802.11 x protocol based wireless card, responses to the stimuli from the individual via touchscreen activations;
▪ measuring reaction times of the responses to the stimuli; and
▪ transmitting, through the 802.11x protocol based wireless card, the received responses and measured reaction times to the assessment computer for storage in a database;
- receive, through the 802.11x protocol based wireless card, cognitive assessment performance data relating to the individual, including the received responses and measured reaction times;
- generate performance metrics including at least a cognitive baseline for the individual based at least on the cognitive assessment performance data, the demographic characteristic data and the environmental data; and
- compare, by the assessment computer, the performance metrics with performance data of other individuals with common demographic characteristics based on the at least one environmental factor.

## Patentansprüche

1. Computergestütztes Verfahren zur Beurteilung der kognitiven Funktion, wobei das Verfahren Folgendes umfasst:
- Empfangen demografischer Daten zu einer Einzelperson an einem Beurteilungscomputer, einschließlich Informationen zum geografischen Standort der tragbaren Berührungsbildschirm-Personalcomputer-Vorrichtung;
- Korrelieren des geografischen Standorts der tragbaren Berührungsbildschirm-Personalcomputer-Vorrichtung mit mindestens einem Umweltfaktor;
- Bereitstellen mindestens eines kognitiven Beurteilungstests an die tragbare Berührungsbildschirm-Personalcomputer-Vorrichtung durch eine erste 802.11x-Protokollkommunikation, der, wenn er durch die tragbare Berührungsbildschirm-Personalcomputer-Vorrichtung ausgeführt wird, die Selbstverwaltung des mindestens einen kognitiven Beurteilungstests durch die Einzelperson ermöglicht durch:
∘ Erzeugen von kognitiven Beurteilungsstimuli, die von der Einzelperson wahrnehmbar sind;
∘ Empfangen von Antworten auf die Stimuli von der Einzelperson über Berührungsbildschirm-Aktivierungen;
∘ Messen der Reaktionszeiten der Antworten auf die Stimuli; und
∘ Übertragen der empfangenen Antworten und gemessenen Reaktionszeiten an den Beurteilungscomputer zum Speichern in einer Datenbank;
- Empfangen von ersten kognitiven Beurteilungsleistungsdaten in Bezug auf die Einzelperson durch eine zweite 802.1 1x-Protokollkommunikation, wobei die erste kognitive Beurteilungsleistung die empfangenen Antworten und die gemessenen Reaktionszeiten einschließt;
- Erzeugen von Leistungsmetriken einschließlich mindestens einer ersten kognitiven Grundlinie für die Einzelperson basierend mindestens auf den ersten kognitiven Beurteilungsleistungsdaten; und
- Vergleichen von mindestens einer ersten kognitiven Grundlinie mit Leistungsdaten anderer Einzelpersonen mit gemeinsamen demografischen Merkmalen auf der Grundlage des mindestens einen Umweltfaktors durch den Beurteilungscomputer.

2. Verfahren nach Anspruch 1, ferner umfassend einen Schritt des Speicherns von Daten in einer Datenbank, auf die der Computer zugreifen kann, die sich auf mindestens eines von Zeitreaktionswerte, Stimuli, Leistungsmetriken, demografische Daten, Tests und Verhalten beziehen.

3. Verfahren nach Anspruch 1, wobei die Stimuli mindestens eines von visuellen Stimuli, Berührungsstimuli und akustischen Stimuli sind.

4. Verfahren nach Anspruch 1, wobei die Messung der Reaktionszeiten auf einem Erscheinungsbild der kognitiven Beurteilungsstimuli und dem Empfangen der Antworten über die Berührungsbildschirm-Aktivierungen basiert.

5. Verfahren nach Anspruch 1, ferner umfassend:
Bereitstellen von Rückmeldung an die Einzelperson in Bezug auf die ersten kognitiven Beurteilungsleistungsdaten.

6. Verfahren nach Anspruch 1, ferner umfassend:
Identifizieren der Einzelperson, wobei das Einzelperson-Benutzerprofil mit einer eindeutigen Kennung der Einzelperson verknüpft ist; und
Zuordnen von ersten kognitiven Beurteilungsleistungsdaten dem Einzelperson-Benutzerprofil.

7. Verfahren nach Anspruch 1, wobei die Erzeugung der Leistungsmetrik einschließt: Speichern der ersten kognitiven Beurteilungsleistungsdaten einschließlich mindestens der Ergebnisse in Bezug auf eine Anzahl von richtig, falsch und nicht beantworteten Fragen, zusammen mit Informationen in Bezug auf die gemessenen Reaktionszeiten.

8. Verfahren nach Anspruch 1, ferner umfassend das Vergleichen der ersten kognitiven Beurteilungsleistungsdaten mit zweiten kognitiven Beurteilungsleistungsdaten von der vorherigen Leistung der Einzelperson, die in der Datenbank gespeichert sind.

9. Verfahren nach Anspruch 1, wobei die erste kognitive Grundlinie automatisch jedes Mal dann aktualisiert wird, wenn die Einzelperson einen kognitiven Beurteilungstest durchführt.

10. Verfahren nach Anspruch 1, wobei die Einzelperson alle demografischen Daten in Bezug auf die Einzelperson selbst meldet.

11. Verfahren nach Anspruch 1, wobei die tragbare Berührungsbildschirm-Personalcomputer-Vorrichtung eine Benutzerleistungsmetrik basierend auf den ersten kognitiven Beurteilungsleistungsdaten erzeugt und einschließlich eines Vergleichs mit der normierten Leistungsmetrik, die auf der Personalcomputer-Vorrichtung gespeichert ist und die Benutzerleistungsmetrik in Echtzeit der Einzelperson anzeigt.

12. Verfahren nach Anspruch 1, wobei die tragbare Berührungsbildschirm-Personalcomputer-Vorrichtung ferner Antworten auf Stimuli von der Einzelperson über mindestens eines von Augenverfolgung und Spracherkennung empfängt.

13. System zur Beurteilung der kognitiven Funktion, wobei das System Folgendes umfasst:
∘ eine auf dem 802.11x-Protokoll basierende Drahtloskarte;
∘ ein tangibles, computerlesbares Medium, das mit einer Einzelperson verbundene kognitive Beurteilungsleistungsdaten, mit der Einzelperson verbundene Umgebungsdaten, demografische Merkmalsdaten und Leistungsmetriken speichert; und
∘ mindestens eine Verarbeitungsvorrichtung, die betrieblich mit dem tangiblen computerlesbaren Medium gekoppelt ist, wobei die mindestens eine Verarbeitungseinheit konfiguriert ist zum:
- Empfangen, an einem Beurteilungscomputer, von demografischen Merkmalsdaten und Umweltdaten;
- Bereitstellen, mittels der auf dem 802.11x-Protokoll basierenden Drahtloskarte, mindestens eines kognitiven Beurteilungstests an eine tragbare Berührungsbildschirm-Personalcomputer-Vorrichtung, der, wenn er durch die tragbare Berührungsbildschirm-Personalcomputer-Vorrichtung ausgeführt wird, die Selbstverwaltung des mindestens einen kognitiven Beurteilungstests durch die Einzelperson ermöglicht durch:
▪ Erzeugen von kognitiven Beurteilungsstimuli, die von der Einzelperson wahrnehmbar sind;
▪ Empfangen von Antworten auf die Stimuli von der Einzelperson über Berührungsbildschirm-Aktivierungen mittels der auf dem 802.11x-Protokoll basierenden Drahtloskarte;
▪ Messen der Reaktionszeiten der Antworten auf die Stimuli; und
▪ Übertragen der empfangenen Antworten und gemessenen Reaktionszeiten an den Beurteilungscomputer zum Speichern in einer Datenbank mittels der auf dem 802.11x-Protokoll basierenden Drahtloskarte;
- Empfangen von kognitiven Beurteilungsleistungsdaten in Bezug auf die Einzelperson mittels der auf dem 802.11x-Protokoll basierenden Drahtloskarte, einschließlich der empfangenen Antworten und der gemessenen Reaktionszeiten;
- Erzeugen von Leistungsmetriken einschließlich mindestens einer kognitiven Grundlinie für die Einzelperson basierend mindestens auf den kognitiven Beurteilungsleistungsdaten, den demografischen Merkmalsdaten und den Umweltdaten; und
- Vergleichen der Leistungsmetriken mit Leistungsdaten anderer Einzelpersonen mit gemeinsamen demografischen Merkmalen auf der Grundlage des mindestens einen Umweltfaktors durch den Beurteilungscomputer.

## Revendications

1. Procédé informatisé d'évaluation de la fonction cognitive, le procédé comprenant :
- la réception, au niveau d'un ordinateur d'évaluation, de données démographiques relatives à un individu, comportant des informations relatives à l'emplacement géographique de dispositif informatique personnel à écran tactile portable ;
- la corrélation de l'emplacement géographique de dispositif informatique personnel à écran tactile portable avec au moins un facteur environnemental ;
- la fourniture, par une première communication selon le protocole 802.11 x, d'au moins un test d'évaluation cognitive sur le dispositif informatique personnel à écran tactile portable, lequel, lorsqu'il est exécuté par le dispositif informatique personnel à écran tactile portable, permet à l'individu d'auto-administrer l'au moins un test d'évaluation cognitive par :
∘ génération de stimuli d'évaluation cognitive perceptibles par l'individu ;
∘ réception de réponses aux stimuli de l'individu par l'intermédiaire d'activations de l'écran tactile ;
∘ mesure des temps de réaction des réponses aux stimuli ; et
∘ transmission des réponses reçues et des temps de réaction mesurés à l'ordinateur d'évaluation pour stockage dans une base de données ;
- la réception, par une seconde communication selon le protocole 802.11x, de premières données de performances d'évaluation cognitive relatives à l'individu, lesdites premières performances d'évaluation cognitive comportant les réponses reçues et les temps de réaction mesurés ;
- la génération de mesures de performances comportant au moins une première base de référence cognitive pour l'individu en fonction au moins des premières données de performances d'évaluation cognitive ; et
- la comparaison, par l'ordinateur d'évaluation, d'au moins une première base de référence cognitive avec des données de performances d'autres individus présentant des caractéristiques démographiques communes en fonction de l'au moins un facteur environnemental.

2. Procédé selon la revendication 1, comprenant en outre une étape de stockage dans une base de données accessible par l'ordinateur des données relatives à au moins l'un de valeurs de temps de réaction, de stimuli, d'indicateurs de performances, de démographie, de tests et de comportement.

3. Procédé selon la revendication 1, dans lequel les stimuli sont au moins l'un de stimuli visuels, de stimuli tactiles et de stimuli audio.

4. Procédé selon la revendication 1, dans lequel la mesure des temps de réaction est fonction de l'apparition des stimuli d'évaluation cognitive et de la réception des réponses par l'intermédiaire des activations de l'écran tactile.

5. Procédé selon la revendication 1, comprenant en outre :
la fourniture d'un retour d'informations à l'individu relatif aux premières données de performances d'évaluation cognitive.

6. Procédé selon la revendication 1, comprenant en outre :
l'identification de l'individu, dans lequel le profil utilisateur de l'individu est lié à un identifiant unique de l'individu ; et
l'association de premières données de performances d'évaluation cognitive au profil utilisateur de l'individu.

7. Procédé selon la revendication 1, dans lequel la génération de mesures de performances comprend : le stockage des premières données de performances d'évaluation cognitive comportant au moins des résultats relatifs à un certain nombre d'interrogations auxquelles il a été répondu correctement, incorrectement et qui sont restées sans réponse, ainsi que des informations relatives aux temps de réaction mesurés.

8. Procédé selon la revendication 1, comprenant en outre la comparaison des premières données de performances d'évaluation cognitive avec des secondes données de performances d'évaluation cognitive provenant des performances précédentes de l'individu, qui sont stockées dans la base de données.

9. Procédé selon la revendication 1, dans lequel la première base de référence cognitive est automatiquement mise à jour chaque fois que l'individu effectue un test d'évaluation cognitive.

10. Procédé selon la revendication 1, dans lequel l'individu déclare lui-même toutes les données démographiques relatives à l'individu.

11. Procédé selon la revendication 1, dans lequel le dispositif informatique personnel à écran tactile portable génère des mesures de performances d'utilisateur en fonction des premières données de performances d'évaluation cognitive et comportant une comparaison avec des mesures de performances normées stockées sur le dispositif informatique personnel, et affiche les mesures de performances d'utilisateur en temps réel à l'individu.

12. Procédé selon la revendication 1, dans lequel le dispositif informatique personnel à écran tactile portable reçoit d'autres réponses aux stimuli de la part de l'individu par l'intermédiaire d'au moins l'un d'un suivi oculaire ou d'une reconnaissance vocale.

13. Système d'évaluation de la fonction cognitive, le système comprenant :
∘ une carte sans fil basée sur le protocole 802.11 x ;
∘ un support tangible lisible par ordinateur stockant des données de performances d'évaluation cognitive associées à un individu, des données environnementales associées à l'individu, des données de caractéristiques démographiques et des mesures de performance ; et
∘ au moins un dispositif de traitement couplé de manière fonctionnelle au support tangible lisible par ordinateur, l'au moins un dispositif de traitement configuré pour :
- recevoir, au niveau d'un ordinateur d'évaluation, les données de caractéristiques démographiques et les données environnementales ;
- fournir, par l'intermédiaire de la carte sans fil basée sur le protocole 802.11x, au moins un test d'évaluation cognitive sur un appareil informatique personnel à écran tactile portable, lequel, lorsqu'il est exécuté par le dispositif informatique personnel à écran tactile portable, permet à l'individu d'auto-administrer l'au moins un test d'évaluation cognitive par :
▪ génération de stimuli d'évaluation cognitive perceptibles par l'individu ;
▪ réception, par l'intermédiaire de la carte sans fil basée sur le protocole 802.11x, de réponses aux stimuli de l'individu par l'intermédiaire d'activations de l'écran tactile ;
▪ mesure des temps de réaction des réponses aux stimuli ; et
▪ transmission, par l'intermédiaire de la carte sans fil basée sur le protocole 802.11x, des réponses reçues et des temps de réaction mesurés à l'ordinateur d'évaluation pour stockage dans une base de données ;
- recevoir, par l'intermédiaire de la carte sans fil basée sur le protocole 802.11x, des données de performance d'évaluation cognitive relatives à l'individu, comportant les réponses reçues et les temps de réaction mesurés ;
- générer des mesures de performances comportant au moins une base de référence cognitive pour l'individu en fonction au moins des données de performances d'évaluation cognitive, des données de caractéristiques démographiques et des données environnementales ; et
- comparer, par l'ordinateur d'évaluation, les mesures de performances avec des données de performances d'autres individus présentant des caractéristiques démographiques communes en fonction de l'au moins un facteur environnemental.
